# EUROPÄISCHE PATENTSCHRIFT

(11) **EP 3 190 955 B1**
(45) Veröffentlichungstag und Bekanntmachung des Hinweises auf die Patenterteilung: **20.11.2019**
(21) Anmeldenummer: 15767312.0
(22) Anmeldetag: 25.08.2015
(51) Int. Cl.: A61B 5/00, A61B 5/0492

(54) **ABLEITELEKTRODE**
RECORDING ELECTRODE
ÉLECTRODE DE DÉRIVATION

(30) Priorität: 12.09.2014 DE 102014113158
(43) Veröffentlichungstag der Anmeldung: 19.07.2017
(73) Patentinhaber: Dr. Langer Medical GmbH, 79183 Waldkirch (DE)
(72) Erfinder: VAN SLYCKE, Sam, NL-8300 Knocke-Heist (NL); LANGER, Andreas, 79183 Waldkirch (DE)
(74) Vertreter: Hennicke, Ernst Rüdiger
(86) Internationale Anmeldenummer: PCT/IB2015/056428
(87) Internationale Veröffentlichungsnummer: WO 2016/038490

(56) Entgegenhaltungen:
- EP-A1- 2 100 559
- WO-A2-2014/048807
- US-A1- 2004 034 396
- US-A1- 2013 023 749

## Beschreibung

Die Erfindung betrifft eine Ableitelektrode zur Ableitung von EMG-Potentialen beim intraoperativen Rekurrenzmonitoring zur Identifikation und Funktionskontrolle des Nervus laryngeus recurrens während eines operativen Eingriffs suchen.

Das intraoperative Neuromonitoring für die Identifikation und Funktionskontrolle des besonders gefährdeten Nervus laryngeus recurrens (Stimmbandenervs) während Eingriffen an der Schilddrüse oder den Nebeschilddrüsen hat sich seit Jahren etabliert. Mittels elektrischer Stimulation über durch den Chirurgen handgeführter Stimulationssonden wird der Nerv bei Berührung erregt. Die Erregung wird über den intakten Nerv zum Zielmuskel (musculus vocalis) weitergeleitet und manifestiert sich hier als Muskelkontraktion, die elektrisch als Elektromyogramm (EMG) abgeleitet wird und ein Summenaktionspotenzial zeigt.

In jüngerer Zeit ist zur intermittierenden Stimulation ein Verfahren zur kontinuierlichen Nervenüberwachung eingeführt worden, bei dem der Nervus vagus über eine permanent in der Gefäß-Nervenscheide um den Nerv gelegte flexible Stimulationselektrode stimuliert wird und dadurch die gesamte Nervenstrecke zu den Stimmbändern hin überwacht. Eine derartige Stimulationselektrode ist z.B. aus der Schrift DE102007036862 A1 bekannt. Eine weitere Elektrodenanordnung gemäß der Präambel des Anspruchs 1 ist in der Schrift US2004034396 A1 offenbart.

Zur Ableitung der Muskelaktionspotenziale von den Stimmbändern können verschiedene Techniken zum Einsatz kommen. Die Verwendung von sogenannten Nadelelektroden bietet durch das Eindringen der Nadelelektrode in das Muskelgewebe den Vorteil sehr gut auswertbarer weitgehend störungsfreier EMG Signale mit vergleichsweise sehr großen Signalamplituden bis hin zu einigen mV. Der Nachteil besteht darin, dass mit solchen Nadelelektroden die Stimmbänder punktiert werden, was das Risiko von Verletzungen, Entzündungen und Hämatomen birgt. Einen Sonderfall der Nadelelektroden stellen bipolar konzentrische Nadelelektroden dar. Eine derartige bipolare Elektrode wird im Situs zwischen Schild- und Ringknorpel eingeführt und nach oben in das jeweilige rechte oder linke Stimmband ohne Sichtkontrolle eingestochen. Dieser Vorgang erfordert Übung und Erfahrung vom Operateur. Abgebrochenen Nadeln und zerstochene Cuffs des Endotrachealtubus mit der Konsequenz, dass eine erneute Intubation des Patienten erforderlich wird, stellen keine Seltenheit dar. Ferner erlaubt diese Technik kein dauerhaftes Verweilen im Situs über die gesamte OP-Zeit.

Es ist auch bekannt, sogenannte "Hooked wire" Elektroden zur Ableitung des Aktionspotentials zu verwenden. Dabei werden hakenförmige Elektroden paarweise per Applikator durch die Trachea unter Sichtkontrolle in die Stimmbänder "eingehakt". Diese Technik hat den Nachteil, dass der zur Atmung verwendete Endotrachealtubus den Zugang zu den Stimmbändern erschwert oder in Einzelfällen unmöglich macht.

In den zurückliegenden Jahren sind die Nadel- und Hakenelektroden aufgrund der Risiken, die mit ihrem Einsatz verbunden sind, mehr und mehr durch Elektroden abgelöst werden, die als in den Tubus eingearbeitete leitfähige Drähte oder Oberflächenelektroden ausgeführt sind, die entweder unmittelbar vor der Intubation des Patienten auf handelsübliche Endotrachealtuben aufgeklebt werden oder als leitfähige Elektrodenstreifen bereits auf den Tubus aufgebracht sind. Bei derartigen Tubuselektroden sind ein- und zweikanalige (EMG) Elektroden bekannt. Hauptproblem sind die durch ungewollte Dislokation des Tubus auch während der Operation gestörten oder gänzlich verschwindenden Signalableitungen. Das kann auch durch die operationsbedingte Drehung der Trachea relativ zum Tubus, der am Mund des Patienten fixiert ist, geschehen. Dennoch gehört diese Technik zu den sichersten und zuverlässigsten Methoden zur EMG Signalableitung von den Stimmbändern. Sollte allerdings eine Ableitung während der Operation über Tubuselektroden nicht erfolgreich sein, beispielsweise, weil bei der Intubation die am Tubus angeordnete Elektrode beschädigt wurde oder aus einem anderen Grund kein Kontakt zwischen den Stimmbändern und der Tubuselektrode hergestellt werden konnte, kann kein Neuromonitoring durchgeführt werden, wenn der Chirurg nicht als Alternative über die Möglichkeit, zur Ableitung eine der oben beschriebenen Nadelelektroden einzusetzen.

Aufgabe der Erfindung ist es, eine Ableitelektrode zu schaffen, die in besonders einfacher Weise durch den Operateur an geeigneter Stelle appliziert werden und während der gesamten OP in der applizierten Lage verbleiben kann, ohne im Operationsfeld (Situs) zu stören, und die beim Monitoring eine sehr gute Signalqualität liefert.

Diese Aufgabe wird mit der Erfindung durch mindestens zwei mittels eines Federelementes relativ zueinander verstellbare, Kontaktbereiche aufweisende oder bildende Hakenelemente gelöst, die unter Wirkung einer Vorspannkraft des Federelementes am präparierten Schildknorpel eines Patienten anklemmbar sind.

Die mindestens zwei mit Kontaktbereichen versehenen Hakenelemente bilden erfindungsgemäß eine bipolare Elektrode, die vom Operateur am Schildknorpel des Patienten, der für die Durchführung der Operation regelmäßig ohnehin freigelegt wird, appliziert werden kann, indem das Federelement vorgespannt wird und sich die Hakenelemente dann unter der Wirkung dieser Vorspannung am Knorpelgewebe anklemmen oder einhaken. Sie erfassen dann die EMG-Signale der unterhalb des Knorpels liegenden Stimmbänder. Es hat sich überraschend gezeigt, dass die so abgeleiteten Signale eine ähnliche und sogar bessere Signalqualität als Nadelelektroden zu liefern im Stande sind. Die Elektrode kann während der gesamten Operation auf dem Schildknorpel fixiert bleiben, wobei sie nach ihrer Applikation durch umgebende Haut abgedeckt werden kann. Sie liegt nicht im unmittelbaren Operationsfeld und stört daher im Situs nicht. Besonders vorteilhaft an der erfindungsgemäßen Ableitelektrode ist, dass sie unter Sichtkontrolle durch den Operateur auf dem Schildknorpel appliziert werden kann, eine exzellente Signalqualität liefert und auch, dass eine Bewegung des Schildknorpels bei Verdrehen der Trachea unkritisch ist, da sich die Stimmbänder des Patienten und die am Schildknorpel unter Vorspannung des Federelements sicher gehaltenen Ableitelektrode immer in gleicher Relativlage zueinander befinden.

In vorteilhafter Weiterbildung der Erfindung kann das Federelement im Wesentlichen von einem elastisch biegsamen Trägerbügel gebildet werden, an dessen Bügelenden die Hakenelemente angeordnet sind. Bei dieser Ausführungsform der Erfindung kommt also eine im Wesentlichen formstabile Konstruktion aus einem elastisch biegsamen Trägermaterial und außen an den Hakenelementen liegenden Elektroden zur Verwendung, die sich krallenartig im gewinkelten Schildknorpel einhaken. Vorzugsweise verläuft der Trägerbügel dabei etwa kreisbogensegment- oder winkelförmig zwischen den bügelendseits angeordneten Hakenelementen und ist mit einem zwischen den Bügelenden angeordneten Druckbereich versehen. Der Druckbereich ist zweckmäßig zumindest annähernd mittig zwischen den Bügelenden angeordnet. Die Der Trägerbügel kann dabei vorzugsweise stärker gewinkelt als der Winkel des Schildknorpels. Um die Ableitelektrode gemäß dieser Ausführungsform am Schildknorpel des Patienten zu applizieren, wird sie von oben auf den Schildknorpel gedrückt, wobei sie sich streckt und die an den Enden befindlichen, zweckmäßig aus Metall bestehenden Haken nach unten freigibt. Die Elektrode ist bestrebt, ihre ursprüngliche Form wieder anzunehmen, wenn sie losgelassen wird. Dabei hakt sie sich so unter der Spannung, die auf sie ausgeübt wurde, beidseitig in den Schildknorpel ein.

Bei einer alternativen Ausgestaltung der Erfindung wird das Federelement in vorteilhafter Weise im Wesentlichen von einem gummielastischen, die in dehnbarem Streifen gebildet, an dessen Streifenenden die Hakenelemente angeordnet sind. Der dehnbare Streifen können vom Operateur auseinandergezogen und hierdurch unter Spannung gesetzt werden, wenn die Elektrode am Schildknorpel des Patienten angesetzt wird. Die Zugspannung in dem Streifen bewirkt dann, dass die Hakenelemente aufeinander zu gezogen werden und sich dabei in dem Knorpelgewebe verkrallen.

Die Hakenelemente oder die daran ausgebildeten Kontaktbereiche sind vorzugsweise über Kabelanschlüsse und/oder über eine Funkstrecke mit einem externen Überwachungsmonitor verbindbar. Die Anschlüsse können dabei einfach mittels zwei Drähten realisiert werden, wobei je ein Anschluss am rechten und ein Anschluss am linken Hakenelement vorgesehen ist. Alternativ kann die Signalübertragung zum Überwachungsmonitor auch als integrierter Funk-ASIC ausgeführt sein, wobei man dann die hierfür erforderlich Funkelektronik beispielsweise an dem Haltebügel anordnen kann.

Wenn das Federelement mit vorzugsweise im Bereich der Hakenelemente angeordneten Ansatzstücken für ein Spannwerkzeug versehen ist, kann es besonders einfach am Schildknorpel appliziert werden. An die Ansatzstücke, die beispielsweise in Form von liegenden Ösen ausgeführt sein können, die sich nahe der äußeren Enden des spannbaren Streifen befinden, kann zur Applikation eine Schere oder Zange angesetzt werden, mit der die Elektrode über dem Schildknorpel gespreizt und mit den nach unten zeigenden Haken auf den Schildknorpel gedrückt wird. Beim anschließenden Loslassen des Werkzeugs krallen sich die Haken infolge der Spannkraft des Federelements in das Gewebe, an dem die Ableitelektrode appliziert wird, bevorzugt also in den Schildknorpel. Das Lösen der Ableitelektrode kann in entsprechend umgekehrter Weise erfolgen.

Weitere Merkmale und Vorteile der Erfindung ergeben sich aus der nachfolgenden Beschreibung und der Zeichnung, worin bevorzugte Ausführungsformen der Erfindung anhand von Beispielen dargestellt und näher erläutert sind. Es zeigt:
- Fig. 1.: den Hals eines Patienten in einer Vorderansicht;
- Fig. 2: den Kehlkopf des Patienten nach Fig. 1 im Querschnitt mit einer ersten Ausführungsform der erfindungsgemäßen Ableitelektrode in vereinfachter Darstellung;
- Fig. 3: die Ableitelektrode gemäß Fig. 2 in einer perspektivischen Darstellung;
- Fig. 4: den Gegenstand der Fig. 3 in einer Draufsicht
- Fig. 5: eine am Kehlkopf des Patienten angelegte zweite Ausführungsform einer erfindungsgemäßen Elektrode in einer Fig. 2 entsprechenden Darstellung;
- Fig.6: die Ableitelektrode gemäß Fig. 5 in schematischer vereinfachter Seitenansicht; und
- Fig. 7: den Gegenstand der Fig. sechs in perspektivischer Darstellung mit daran angesetzten Applikationswerkzeug.

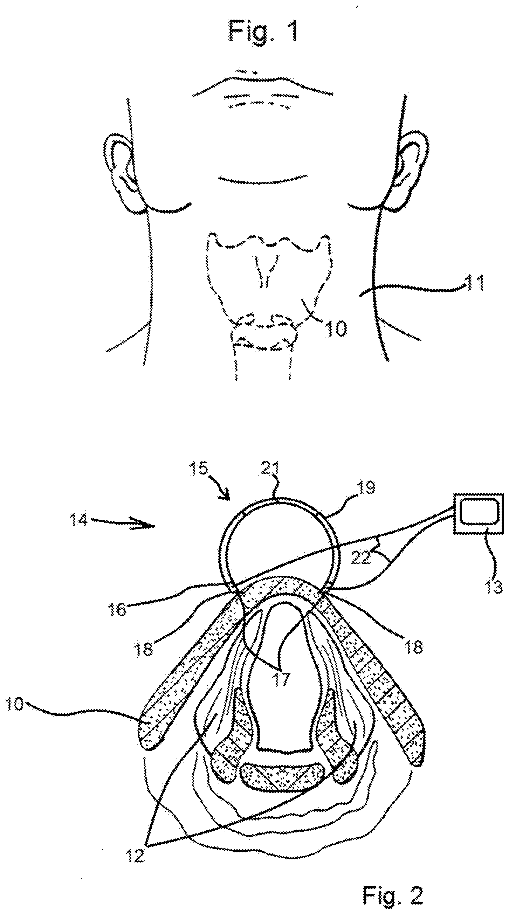

Die Fig. 1 zeigt einen Patienten mit nach oben verschwenktem Kinn, um die Lage des mit 10 bezeichneten Schildknorpels darzustellen, der den Kehlkopf im Hals 11 definiert, wo die Stimmbänder angeordnet sind.

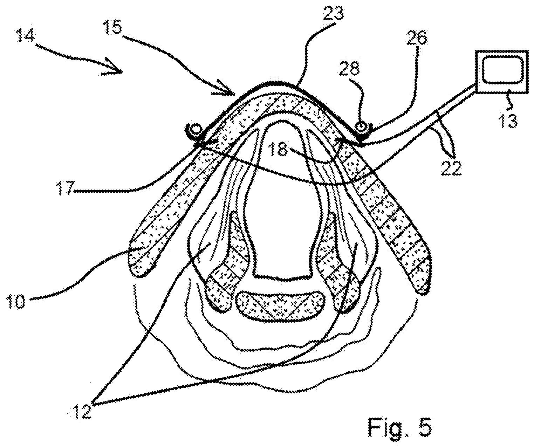

Die die Figuren 2 und 5 zeigen im Querschnitt den während einer Operation freipräparierten Schildknorpel 10 mit den darunter verlaufenden Stimmbandmuskeln 12 (musculi vocali). Die Stimmbänder 12 stellen die Zielmuskeln beim intraoperativen Neuromonitoring des Nervus laryngeus recurrens (Stimmbandnerv) dar, der vom Operateur bei einem Eingriff an der Schilddrüse während der Operation mittels einer hier nicht dargestellten Stimulationselektrode erregt wird, um seine Funktionsfähigkeit zu überprüfen. Die Erregung wird über den intakten Nerv zu den Stimmbändern 12 weitergeleitet und manifestiert sich hier als Muskelkontraktion, die elektrisch als Elektromyogramm (EMG) abgeleitet wird und die an einem Monitor 13 ein Summenaktionspotenzial zeigt.
Für die Ableitung des an den Muskeln auftretenden Potenzials dient eine Ableitelektrode 14, die Gegenstand der vorliegenden Erfindung ist und die am bei der Operation freigelegten Schildknorpel 10 appliziert wird. Hierzu weist die erfindungsgemäße Ableitelektrode 14 ein elastisches Federelement 15 auf, an dessen Enden 16 Hakenelemente 17 ausgebildet sind, die Kontaktbereiche 18 bilden. Unter der Wirkung der Federkraft nach Vorspannung des Federelements 15 werden die Hakenelemente 17 mit den daran ausgebildeten Kontaktbereichen 18 am Knorpelgewebe des präparierten Schildknorpels 10 angeklemmt, wie dies insbesondere in den Fig. 2 und 5 erkennbar ist.

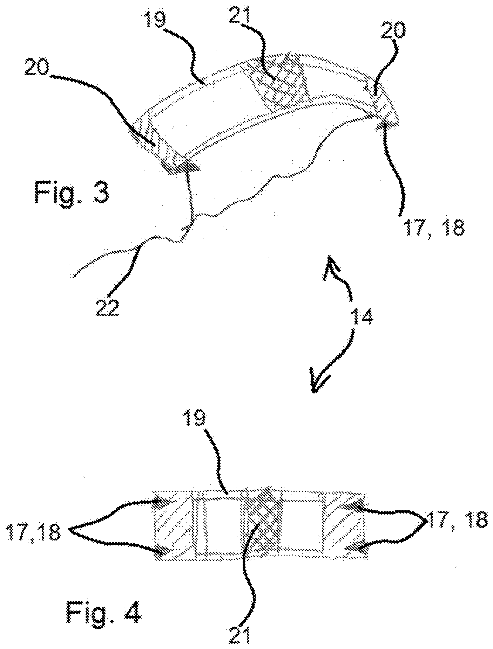

Bei der in den Figuren 2 bis 4 dargestellten, ersten Ausführungsform der erfindungsgemäßen Elektrode 14 wird das Federelement 15 von einem elastisch biegsamen Trägerbügel 19 gebildet, an dessen freien Bügelenden 20 die Hakenelemente 17 angeordnet sind. Der Trägerbügel 19 verläuft zwischen den bügelendseits vorgesehenen Hakenelementen 17 etwa kreisbogensegmentförmig und ist mit einem mittig zwischen den Bügelenden vorgesehenen Druckbereich 21 versehen. Zur Applikation dieser Ableitelektrode am Schildknorpel 10 wird sie vom Operateur einfach mit den Bügelenden auf das Knorpelgewebe aufgesetzt und dann durch Druck auf den Druckbereich 21 gegen den Schildknorpel 10 gedrückt. Hierdurch werden die Bügelenden 20 seitlich auseinandergedrückt und der Bügel 19 kommt unter Spannung. Beim Loslassen des Druckbereiches dann fassen die nach innen weisenden Hakenelemente 17 an den Bügelenden 20 widerhakenartig in das Knorpelgewebe des Schildknorpels 10 und krallen sich in diesem fest, wobei die Kontaktbereiche 18, die bis in die Spitzen der Hakenelemente 17 reichen, ausreichend tief in das Gewebe einfassen und einen sicheren Kontakt mit diesem gewährleisten. Die Ableitung der bei der Kontraktion der Stimmbandmuskeln 12 im Schildknorpel hervorragend feststellbaren Signale erfolgt über Anschlusskabel 22, die fest an den Kontaktbereichen 18 angeschlossen sind und die mit dem Monitor 13 verbunden sind/werden.

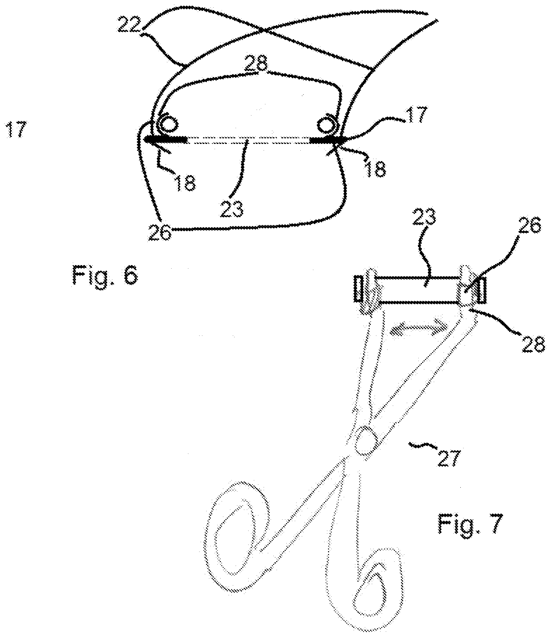

Ähnlich ist das Prinzip bei der zweiten Ausführungsform der Ableitelektrode 14, wie sie in den Fig. 5 bis 7 dargestellt ist. Hier wird das Federelement 15 von einem gummielastischen, dehnbaren Streifen 23 gebildet, an dessen Streifenenden 24 die Hakenelemente 17 angeordnet sind. Um diese Bauart der erfindungsgemäßen Ableitelektrode in besonders einfacher Weise am Schildknorpel anbringen zu können, sind an der dem Schildknorpel 10 abgewandten Oberseite 25 des Streifens im Bereich der Hakenelemente 17 Ansatzstücke 26 für ein Spannwerkzeug 27 vorgesehen, von dem in Fig.5 lediglich im Querschnitt kreisförmig erscheinende Spreizbacken 28 sichtbar sind. Das Spannwerkzeug 27, das wie in Fig. 7 dargestellt scherenartig bzw. nach Art einer Sprengringzange gestaltet sein kann, wird mit den Spreizbacken 28 an den Ansatzstücken 26 angesetzt, so dass beim Öffnen des Werkzeugs 27 dessen Spreizbacken 28 auseinanderbewegt werden und hierdurch den gummielastischen Streifen 23 dehnen. Mit dem Werkzeug kann die Ableitelektrode 14 dann von oben auf den Schildknorpel 10 aufgesetzt werden, wobei die endseitig am Streifen angeordneten Hakenelemente 17 an den beiden Seiten des Knorpels 10 in Anlage kommen und dann beim Lösen des Werkzeugs 28 von dem gummielastischen, vorgespannten Streifen 23 schräg nach oben gezogen werden und sich dabei in das Knorpelgewebe hineinbohren. Die an den Hakenspitzen ausgebildeten Kontaktbereiche 18 bleiben während der gesamten Applikationsdauer sicher in Kontakt mit dem Gewebe und gewährleisten - auch hier über Anschlusskabel 22 - eine zuverlässige Ableitung der elektrischen Signale, die bei einer Kontraktion der unter dem Schildknorpel verlaufenden Stimmbänder 11,12 auftreten. Zum Abnehmen der Ableitelektrode kann wiederum das Spannwerkzeug eingesetzt werden, um den Streifen, 23 zu dehnen und hierdurch die Hakenelemente außer Eingriff mit dem Knorpelgewebe des Schildknorpels zu bringen. Falls es sich bei der Elektrode um ein nur zum einmaligen Gebrauch bestimmtes Teil handelt, kann man den elastischen Streifen auch einfach mit einer Schere durchtrennen, was dann sicher gewährleistet, dass die Elektrode kein zweites Mal verwendet werden kann.

Die Erfindung ist nicht auf die dargestellten und beschriebenen Ausführungsbeispiele beschränkt. Beispielsweise ist es möglich, die Übertragung der von der Ableitelektrode festgestellten Signale alternativ zu den Kabeln 22 über eine Funkstrecke zu realisieren. In diesem Fall kann der erforderliche (Miniatur-)Funksender am Trägerbügel 19 beispielsweise unterhalb des Druckbereichs angeordnet sein oder an dem elastischen Streifen 23 zwischen den Ansatzstücken für das Spannwerkzeug angeklebt, angenäht oder in anderer geeigneter Weise befestigt sein.

## Patentansprüche

1. Ableitelektrode zur Ableitung von EMG-Potentialen beim intraoperativen Rekurrenzmonitoring zur Identifikation und Funktionskontrolle des Nervus laryngeus recurrens während eines operativen Eingriffs, **gekennzeichnet durch** mindestens zwei mittels eines Federelements (15) relativ zueinander verstellbare, Kontaktbereiche (18) aufweisende oder bildende Hakenelemente (17), die unter Wirkung einer Vorspannkraft des Federelements (15) am präparierten Schildknorpel (10) eines Patienten anklemmbar sind.

2. Ableitelektrode nach Anspruch 1, **dadurch gekennzeichnet, dass** das Federelement (15) im Wesentlichen von einem elastisch biegsamen Trägerbügel (19) gebildet wird, an dessen Bügelenden (20) die Hakenelemente (17) angeordnet sind.

3. Ableitelektrode nach Anspruch 1 oder 2, **dadurch gekennzeichnet, dass** der Trägerbügel (19) etwa kreisbogensegment- oder winkelförmig zwischen den bügelendseitig angeordneten Hakenelementen (17) verläuft und mit einem zwischen den Bügelenden angeordneten Druckbereich (21) versehen ist.

4. Ableitelektrode nach Anspruch 3, **dadurch gekennzeichnet, dass** der Druckbereich (21) zumindest annähernd mittig zwischen den Bügelenden (20) angeordnet ist.

5. Ableitelektrode nach Anspruch 1, **dadurch gekennzeichnet, dass** das Federelement (15) im Wesentlichen von einem gummielastischen, dehnbaren Streifen (23) gebildet wird, an dessen Streifenenden (24) die Hakenelemente (17) angeordnet sind.

6. Ableitelektrode nach einem der Ansprüche 1 bis 5, **dadurch gekennzeichnet, dass** die Hakenelemente (17) oder die daran ausgebildeten Kontaktbereiche (18) über Anschlusskabel (22) und/oder über eine Funkstrecke mit einem externen Überwachungsmonitor (13) verbindbar sind.

7. Ableitelektrode nach einem der Ansprüche 1 bis 6, **dadurch gekennzeichnet, dass** das Federelement (15) mit vorzugsweise im Bereich der Hakenelemente (17) angeordneten Ansatzstücken (26) für ein Spannwerkzeug (27) versehen ist.

## Claims

1. Recording electrode for recording EMG potentials during intraoperative recurrence monitoring for identification and functional control of the recurrent laryngeal nerve during a surgical procedure, **characterised by** at least two hook elements (17) which can be adjusted relative to each other using a spring element (15) and have or form contact areas (18) and can be clamped under the effect of a prestressing force of the spring element (15) on the prepared thyroid cartilage (10) of a patient.

2. Recording electrode according to claim 1, **characterised in that** the spring element (15) is substantially formed by an elastically flexible support bracket (19), on whose bracket ends (20) the hook elements (17) are arranged.

3. Recording electrode according to claim 1 or 2, **characterised in that** the support bracket (19) extends approximately in a circular arc segment or angularly between the hook elements (17) arranged on the bracket ends and is provided with a pressure area (21) arranged between the bracket ends.

4. Recording electrode according to claim 3, **characterised in that** the pressure area (21) is arranged at least approximately centrally between the bracket ends (20).

5. Recording electrode according to claim 1, **characterised in that** the spring element (15) is substantially formed by a rubber-elastic, stretchable strip (23), on the strip ends (24) of which the hook elements (17) are arranged.

6. Recording electrode according to any of claims 1 to 5, **characterised in that** the hook elements (17) or the contact areas (18) formed thereon can be connected via connecting cables (22) and/or via a radio link to an external monitor (13).

7. Recording electrode according to any of claims 1 to 6, **characterised in that** the spring element (15) is provided with extension pieces (26) for a clamping tool (27), said extension pieces being preferably arranged in the area of the hook elements (17).

## Revendications

1. Electrode de dérivation pour dériver des potentiels d'EMG lors du monitoring récurrentiel intra-opératoire pour l'identification et le contrôle des fonctions du nerf récurrent pendant une intervention chirurgicale, **caractérisée par** au moins deux éléments d'accrochage (17) présentant ou formant des zones de contact (18), réglables l'un par rapport à l'autre avec au moins un élément élastique (15), qui peuvent être fixés au cartilage thyroïde (10) préparé d'un patient sous l'effet d'une force de précontrainte de l'élément élastique (15).

2. Electrode de dérivation selon la revendication 1, **caractérisée en ce que** l'élément élastique (15) est formé sensiblement par un étrier porteur (19) flexible élastiquement aux extrémités d'étrier (20) duquel les éléments d'accrochage (17) sont disposés.

3. Electrode de dérivation selon la revendication 1 ou 2, **caractérisée en ce que** l'étrier porteur (19) évolue à peu près sous la forme de segment d'arc de cercle ou sous une forme angulaire entre les éléments d'accrochage (17) disposés au niveau de l'extrémité d'étrier et est doté d'une zone de pression (21) disposée entre les extrémités d'étrier.

4. Electrode de dérivation selon la revendication 3, **caractérisée en ce que** la zone de pression (21) est disposée au moins approximativement au milieu entre les extrémités d'étrier (20).

5. Electrode de dérivation selon la revendication 1, **caractérisée en ce que** l'élément élastique (15) est formé sensiblement par des bandes étirables (23) en caoutchouc élastique aux extrémités de bande (24) desquelles les éléments d'accrochage (17) sont disposés.

6. Electrode de dérivation selon l'une quelconque des revendications 1 à 5, **caractérisée en ce que** les éléments d'accrochage (17) ou les zones de contact (18) formées sur ceux-ci peuvent être reliés par câble de raccordement (22) et/ou par une voie radioélectrique avec un moniteur de surveillance (13) externe.

7. Electrode de dérivation selon l'une quelconque des revendications 1 à 6, **caractérisée en ce que** l'élément élastique (15) est doté d'embouts (26) disposés de préférence dans la zone des éléments d'accrochage (17) pour un outil de serrage (27).
